# EUROPEAN PATENT APPLICATION

(11) **EP 1 582 163 A1**
(43) Date of publication of application: **05.10.2005**
(21) Application number: 04007789.3
(22) Date of filing: 31.03.2004
(51) Int. Cl.: A61B 17/72

(54) **Intramedullary nail comprising an helical element of shape-memory material**

(71) Applicant: ORTHOFIX INTERNATIONAL B.V., 1071 Amsterdam (NL)
(72) Inventor: Coati, Michele, 37029 San Pietro in Cariano (Verona) (IT); Marazzi, Giancarlo, 21047 Saronno (Varese) (IT); Marini, Graziano, 37060 Castel D'Azzano (Verona) (IT); Rossi, Graziano, 37139 Verona (IT); Rossi, Luigi, 37019 Peschiera de Garda (Verona) (IT); Venturini, Daniele, 37064 Povegliano Veronese (Verona) (IT)
(74) Representative: Ferreccio, Rinaldo

(57) **Abstract**

An intramedullary nail (10, 110) suitable for insertion in a fractured elongate bone (12) and for an unusually simple fixation therein comprises a substantially straight stem (14), having a predetermined axis (X-X), extending between a proximal end (16) and a distal end (18), and an helical element (20) realised with a shape-memory material, said helical element (20) being positioned along said axis (X-X) and elongation-constraint means (21) being provided in correspondence with said proximal (16) and distal (18) ends, said helical element (20) elongating in the helical development thereof so that coils thereof (22) are positioned outwards said axis (X-X) for the nail fixation to the bone.

## Description

### Field of application

The present invention relates in its more general aspect to an intramedullary nail suitable for insertion in a fractured elongate bone and an application method of said nail in said bone.

In particular, the invention relates to an intramedullary nail suitable for insertion in a fractured elongate bone, such as a femur or a tibia, comprising a substantially straight stem, having a predetermined axis, extending between a proximal end and a distal end.

### Prior art

Intramedullary nails are known, which, during a surgical operation, are inserted in a fractured elongate bone and fixed therein, in order to reconstruct the original bone configuration and meanwhile recover the bone solidity, so that callus regeneration mechanisms can correctly occur.

The stems of these intramedullary nails are generally cylinder-shaped and they can be both solid and hollow.

In order to fix the intramedullary nail to the bone portions to be reconstructed, two offset holes are usually provided on the nail, having axes lying on parallel planes and extending diametrically across the stem, in correspondence with the nail distal end, and two offset holes having the same size, having axes not necessarily lying on parallel planes, in correspondence with the nail proximal end. Said holes are suitable for housing bone screws, which are inserted, after a convenient bone drilling, in the bone, with the subsequent fixation of the intramedullary nail to the bone portions.

Although advantageous under different points of view, intramedullary nails being structured as above schematically described have known drawbacks mainly underlined when bone drillings are to be performed for bone screw insertion. This step is particularly critical since it is known that a good nail fastening essentially depends on the correct realisation of these bone drillings, obviously made in correspondence with the holes of the inserted intramedullary nail.

However the precise location of the intramedullary nail holes is made difficult by the fact that the holes are no more visible, being the nail inserted in the bone. It is then worth underlining a further location problem, i.e. the fact that the intramedullary nail can be, when being inserted, slightly bent, so that the holes at the nail distal end are no more, with respect to the proximal end, in the same position as before installing the nail.

The traditional technique for locating the holes of an inserted intramedullary nail provides the use of X rays, involving however the well known risks of cumulative exposure of the operating staff, besides being quite awkward during the surgical operation.

Specific mechanical devices have thus been studied and realised, such as for example the one described in the European patent no. EP 772 420 in the name of the same Applicant.

These devices do not require the use of X rays, but they have the drawback of requiring several operational steps, all to be performed quite carefully and precisely.

### Summary of the invention

The problem underlying the present invention is to provide an intramedullary nail suitable for insertion in a fractured elongate bone, capable to meet the above-mentioned requirement, meanwhile overcoming, in a simple and effective way, all the drawbacks mentioned with reference to the prior art.

This problem is solved, according to the present invention, by an intramedullary nail suitable for insertion in a fractured elongate bone, as above described and characterised in that it comprises an helical element realised with a shape-memory material, said helical element being positioned along said axis and elongation-constraint means being provided in correspondence with said proximal and distal ends, said helical element elongating in the helical development thereof so that coils thereof are positioned outwards said axis for the nail fixation to the bone.

Further features and the advantages of the intramedullary nail suitable for insertion in a fractured elongate bone, as well as of the application method of said nail in said fractured bone, according to the present invention, will be apparent from the following description of an embodiment thereof made with reference to the attached drawings, given by way of non-limiting example.

### Brief description of the drawings

Figure 1 is a schematic perspective view of an intramedullary nail, suitable for insertion in a fractured elongate bone, according to the present invention.
Figure 2 is a schematic perspective exploded view of the nail of figure 1.
Figure 3 is a schematic perspective view of an alternative embodiment of an intramedullary nail according to the present invention.

### Detailed description

With reference to the drawings, an intramedullary nail according to the present invention is shown in a first embodiment and in an alternative embodiment.

The nail, in the embodiment described at first, is globally indicated with 10, while in the alternative embodiment the nail of the invention is indicated with 110. It is specified that, in this alternative embodiment, the elements being structurally or functionally similar to the elements of the nail 10 are indicated with the same reference number and the detailed description thereof is not repeated for short.

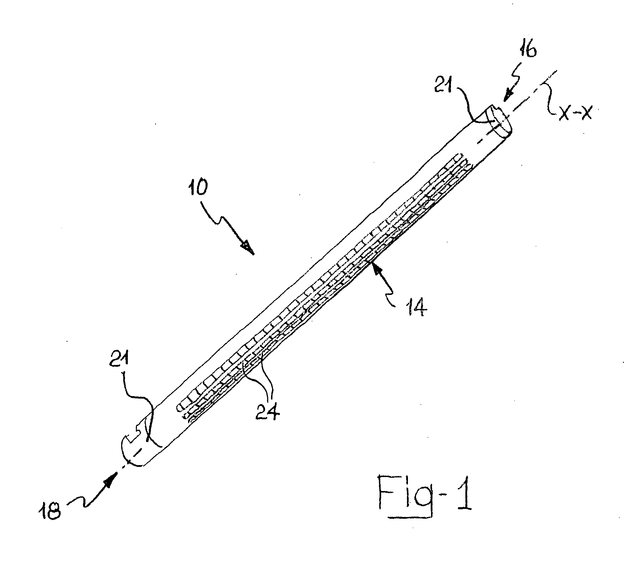

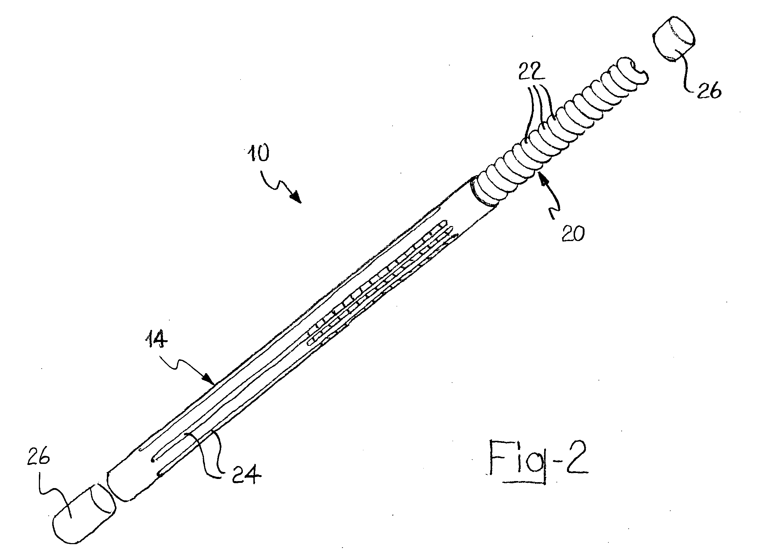

The nail 10 shown in figures 1 and 2, which is suitable for insertion in a fractured elongate bone 12, such as for example a femur or a tibia, comprises a substantially straight stem 14, having a predetermined X-X axis, extending between a proximal end 16 and a distal end 18. The stem 14 is preferably composed of a cylindrical tubular body.

According to an aspect of the present invention, the nail 10 comprises an helical element 20 realised in a shape-memory material, said helical element 20 being positioned along said axis X-X and elongation-constraint means 21 being provided in correspondence with said proximal 16 and distal 18 ends, said helical element 20 elongating in the helical development thereof so that coils thereof 22 are positioned outwards said axis X-X for the nail fixation to the bone.

Shape memory material means a material having a given initial starting shape and taking, under predetermined external conditions (for example a temperature rise and/or drop) or undergoing a predetermined activation condition, i.e. after a so-called "material instruction" step, a given new shape.

Known shape-memory materials, which are suitable for use in the present invention, are, for instance, certain nickel-titanium alloys.

The invention is based on the following principle: subjecting an element realised in a shape memory material, having a predetermined initial shape at rest, to said so-called "material instruction" step (for example to a predetermined temperature variation), said element takes a shape being maintained as long as the "instruction" step effect persists; said shape, being different from the initial shape, can be called transient shape and it is temporary or unstable. When the "instruction" step effect stops, the element leaves said transient shape and, coming back towards the initial shape, takes a working shape. Depending on the type of material and on the working temperature, in the working shape, said material can arrive at the initial shape or it can go further on the initial shape, arriving at a final shape. It is worth pointing out that the more this final shape is far from the initial shape, the more the available shape memory energy of the material is high.

The invention has been reached as result of the intuition that an element realised in a shape memory material, in the passage from the transient shape towards the working shape, can create the fixing of the intramedullary nail in the bone.

In the embodiment of figures 1 and 2, the helical element 20, for example with circular cross-section, is positioned in the cylindrical tubular body of the stem 14.

More precisely, the stem 14 is elastically deformable in a direction being substantially radial to the axis X-X. Preferably, a plurality of longitudinal slots 24, developed for example for most of the length of the stem 14 and angularly equally spaced, are provided around the cylindrical tubular body.

In correspondence with each of the two proximal 16 and distal 18 ends, said elongation-constraint means 21 shape in a closing element 26 which is connected to the cylindrical tubular body of the stem 14: said closing element 26 closes the central hole of the cylindrical tubular body of the stem 14. Preferably, the closing element 26 is cylinder-shaped.

In the initial shape, the helical element 20 is inserted in the cylindrical tubular body of the stem 14 and it has a longitudinal development being lower or substantially equal to the distance between the two closing elements 26. The radial dimensions of the coils 22 or the helical element 20 are such as to be comprised in the cylindrical tubular body of the stem 14, without causing a deformation thereof.

In the final shape, i.e. when the shape-memory material of the helical element 20 is activated, the helical element 20 is elongate: being however constrained to the longitudinal elongation by the constraint means 21, it results that the radial dimensions of the coils 22 increase and consequently the cylindrical tubular body of the stem 14 is radially expanded.

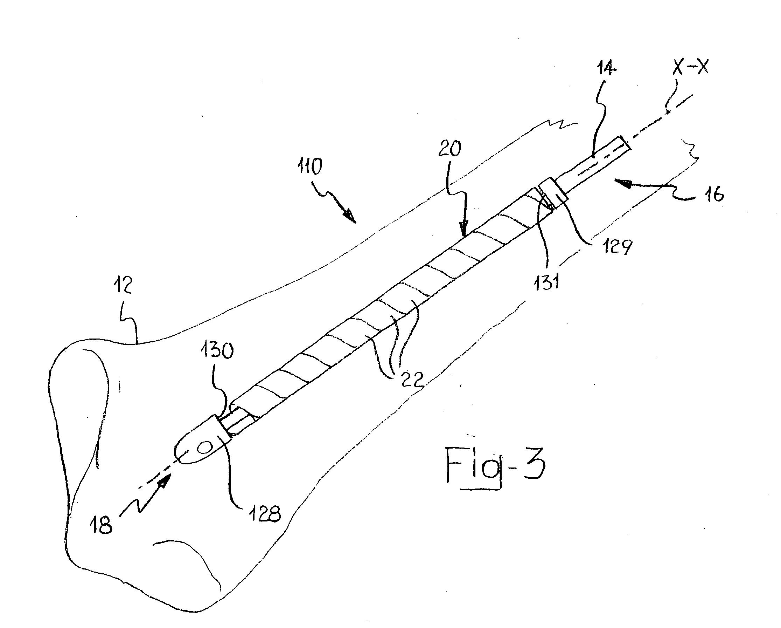

In the alternative embodiment of figure 3, the nail 110 according to the invention is shown, wherein the helical element 20, for example with quadrilateral cross-section, is positioned outwards the stem 14.

More precisely, the stem 14 is composed of a cylindrical tubular body.

Said elongation-constraint means 21 in correspondence with an end of the stem 14, for example in correspondence with the distal end 18, shape in an enlargement 128 of the stem 14. The enlargement 128 is equipped, on the side towards the centre of the stem 14, with a beat collar 130 for an end of the helical element 20. Preferably, the enlargement 128 is tapered on the opposite side with respect to the beat collar 130, to help in inserting the nail 110.

At the opposite end, i.e. for example at the proximal end, the elongation-constraint means 21 shape in a further enlargement 129 of the stem 14. Similarly to the enlargement 128, the enlargement 129 is equipped, on the side towards the centre of the stem 14, with a beat collar 131 for the opposite end of the helical element 20. Preferably, the enlargement 129 is cylinder-shaped.

In the initial shape, the helical element 20 is put around the cylindrical tubular body of the stem 14 and it has a longitudinal development being lower or substantially equal to the distance between the enlargement 128 and the further enlargement 129.

In the final shape, i.e. when the shape-memory material of the helical element 20 is activated, the helical element 20 is elongate: being however constrained to the longitudinal elongation by the constraint means 21, it results that the radial dimensions of the coils 22 increase.

An application method of an intramedullary nail according to the present invention in said fractured elongate bone 12 comprises:
a location step of said nail in said elongate bone 12, to mend the fracture;
an activation step of said helical element 20 wherein the coils thereof 22 are positioned outwards said axis X-X for the nail fixation to the bone, said helical element 20 being realised with a shape-memory material.

The operation of the intramedullary nail, suitable for insertion in a fractured elongate bone 12, according to the present invention, is already partially evident from the description of the above application method and it is specified hereafter.

The nail according to the invention is positioned in said elongate bone 12, in the initial shape thereof.

Afterwards, in order to fix the nail to the bone 12, said helical element 20 realised with a shape-memory material is activated: the coils 22 of said helical element 20 tend to be positioned outwards the axis X-X of the stem 14.

In the first embodiment, the enlarging coils 22 tend to radially expand the cylindrical tubular body of the stem 14 to realise an interference between said tubular body and the bone portion surrounding it. Evidently, the cylindrical tubular body expansion is favoured by the slots 24.

This interference causes in practise a grip, solidly fixing the nail in the bone 12.

It must be noticed that the expansion of the cylindrical tubular body is of the elastic type: this favours a possible removal operation of the nail from the bone wherein it has been fixed

In the alternative embodiment, the enlarging coils 22 realise an interference with the bone portion surrounding them. This interference causes in practise a grip, solidly fixing the nail in the bone 12.

The main advantage achieved by the intramedullary nail suitable for insertion in a fractured elongate bone, as well as by the application method of said nail in said fractured bone, according to the present invention, is the fact of unusually simplifying the fixation step of the intramedullary nail inserted in the fractured bone. In practise, the nail fixation is obtained simultaneously with the insertion thereof in the bone, since the shape-memory elements, cooled at first to take the initial shape, take again in the bone their final shape, due to the heat released by the patient's body.

Another advantage of the intramedullary nail according to the present invention, is to prevent undesired nail torsions or rotations in the bone during the application step.

Obviously, in order to meet specific and contingent requirements, a skilled in the art could bring several changes to the above-described intramedullary nail suitable for insertion in a fractured elongate bone and application method of said nail in said bone, all however comprised in the scope of protection of the present invention, as defined in the following claims.

## Claims

1. Intramedullary nail (10, 110) suitable for insertion in a fractured elongate bone (12), comprising a substantially straight stem (14), having a predetermined axis (X-X), extending between a proximal end (16) and a distal end (18), **characterised in that** it comprises an helical element (20) realised with a shape-memory material, said helical element (20) being positioned along said axis (X-X) and elongation-constraint means (21) being provided in correspondence with said proximal (16) and distal (18) ends, said helical element (20) elongating in the helical development thereof so that coils thereof (22) are positioned outwards said axis (X-X) for the nail fixation to the bone.

2. Intramedullary nail (10, 110) according to claim 1, **characterised in that** the stem (14) is composed of a cylindrical tubular body.

3. Intramedullary nail (10) according to claim 2, **characterised in that** the helical element (20) is positioned in the cylindrical tubular body of the stem (14).

4. Intramedullary nail (10) according to claim 2, **characterised in that** the helical element (20) has a circular cross-section.

5. Intramedullary nail (10) according to claim 3, **characterised in that** the stem (14) is elastically deformable in a substantially radial direction with respect to said axis (X-X).

6. Intramedullary nail (10) according to claim 5, **characterised in that** a plurality of longitudinal slots (24) are provided around the cylindrical tubular body of the stem (14).

7. Intramedullary nail (10) according to claim 6, **characterised in that** the longitudinal slots (24) are developed for most of the length of the stem (14) and they are angularly equally spaced.

8. Intramedullary nail (10) according to claim 3, **characterised in that** , in correspondence with each of the two proximal (16) and distal (16) ends, said elongation-constraint means (21) shape in a closing element (26) which is connected to the cylindrical tubular body of the stem (14) and which closes the central hole of the cylinder tubular body of the stem (14).

9. Intramedullary nail (10) according to claim 8, **characterised in that** the closing element (26) is cylinder-shaped.

10. Intramedullary nail (10) according to claim 8, **characterised in that**, in the shape preceding the nail fixation, the helical element (20) has a longitudinal development being lower or substantially equal to the distance between the two closing elements (26).

11. Intramedullary nail (10) according to claim 10, **characterised in that** the radial dimensions of the coils (22) of the helical element (20) are such as to be comprised in the cylindrical tubular body of the stem (14).

12. Intramedullary nail (10) according to claim 10, **characterised in that**, when the shape-memory material of the helical element (20) is activated, the helical element (20) is elongate.

13. Intramedullary nail (110) according to claim 1, **characterised in that** the helical element (20) is positioned outwards the stem (14).

14. Intramedullary nail (110) according to claim 13, **characterised in that** the helical element (20) has a quadrilateral cross-section.

15. Intramedullary nail (110) according to claim 13, **characterised in that** said elongation-constraint means (21) in correspondence with an end (18) of the stem (14) shape in an enlargement (128) of the stem (14).

16. Intramedullary nail (110) according to claim 13, **characterised in that** the enlargement (128) is equipped, on the side towards the centre of the stem (14), with a beat collar (130) for an end of the helical end (20).

17. Intramedullary nail (110) according to claim 16, **characterised in that** the enlargement (128) is tapered on the opposite side with respect to the beat collar (130).

18. Intramedullary nail (110) according to claim 15, **characterised in that** at the opposite end (16), the elongation-constraint means (21) shape in a further enlargement (129) of the stem (14).

19. Intramedullary nail (110) according to claim 18, **characterised in that** the further enlargement (129) is equipped, on the side towards the centre of the stem (14), with a beat collar (131) for the opposite end of the helical element (20).

20. Intramedullary nail (110) according to claim 18, **characterised in that** the further enlargement (129) is cylinder-shaped.

21. Intramedullary nail (110) according to claim 18, **characterised in that**, in the shape preceding the nail fixation, the helical element (20) is put arount the cylindrical tubular body of the stem (14) and it has a longitudinal development being lower or substantially equal to the distance between the enlargement (128) and the further enlargement (129).

22. Intramedullary nail (10) according to claim 21, **characterised in that**, when the shape-memory material of the helical element (20) is activated, the helical element (20) is elongate.

23. Intramedullary nail (10, 110) according to claim 1, **characterised in that** the fractured elongate bone (12) is a femur or a tibia.

24. Application method, in a fractured elongate bone (12), of an intramedullary nail (10, 110) according to claim 1, **characterised in that** it comprises:
a location step of said nail (10, 110) in said elongate bone (12), to mend the fracture;
an activation step of said helical element (20) wherein the coils thereof (22) are positioned outwards said axis (X-X) for the nail fixation to the bone, said helical element (20) being realised with a shape-memory material.
